# FASCICULE DE BREVET EUROPEEN

(11) **EP 0 430 750 B1**
(45) Date de publication et mention de la délivrance du brevet: **25.01.1995**
(21) Numéro de dépôt: 90403231.5
(22) Date de dépôt: 15.11.1990
(51) Int. Cl.: G01N 33/50

(54) **Réactif et méthode d'utilisation de celui-ci pour la détermination automatique en cytométrie de flux d'au moins une sous-population leucocytaire à partir du sang total**
Reagenz und Verfahren zur Verwendung desselben in der automatischen durchflusszytometrischen Bestimmung von mindestens einer Leukocyten-Subpopulation aus Vollblut
Reagent and its use in flow cytometry for automatically determining at least one leukocyte subpopulation obtained from whole blood

(30) Priorité: 20.11.1989 FR 8915166
(43) Date de publication de la demande: 05.06.1991
(73) Titulaire: ABX , Société Anonyme dite, F-34100 Montpellier (FR)
(72) Inventeur: Lefèvre Didier, F-78200 Mantes la Ville (FR); Champseix Henri, F-78360 Montesson (FR); Voltas Nadine, F-33000 Bordeaux (FR)
(74) Mandataire: Lhuillier, René

(56) Documents cités:
- EP-A- 0 214 613
- WO-A-84/04969
- WO-A-85/05684
- GB-A- 2 077 916
- US-A- 4 286 963
- AMERICAN JOURNAL OF CLINICAL PATHOLOGY, 1981, American Society of Clinical Pathologists; L. KASS, pp. 810-812/

## Description

L'invention a pour objet l'analyse de formules sanguines par comptage et discrimination de sous-populations leucocytaires et concerne plus particulièrement un réactif et une méthode d'utilisation de celui-ci pour la détermination, en cytométrie de flux, d'au moins une sous-population leucocytaire.

L'importance diagnostique de la détermination précise des différentes populations leucocytaires est reconnue depuis longemps. En effet, l'apparition de rapports leucocytaires anormaux peut être corrélée à l'apparition de certaines maladies (réactions immunitaires, réactions inflammatoires, apparition de néoplasmes ou de leucémies. ..).

Les méthodes traditionnelles d'analyse manuelle comprenant une séparation physique des érythrocytes (par sédimentation ou aggrégation) puis des colorations différentielles des cytoplasmes et des noyaux des leucocytes et leur observation morphologique au microscope, permettaient déjà d'identifier tous les types de leucocytes c'est à dire les granulocytes (basophiles, éosinophiles et neutrophiles) et les agranulocytes (monocytes et lymphocytes). Ces méthodes donnent des résultats fiables mais leur mise en oeuvre est longue, ce qui les rend rarement ou difficilement transposables aux déterminations en appareils automatiques.

Différents appareils sont déjà commercialisés qui permettent le comptage automatique des populations leucocytaires et divers réactifs et colorants ont été mis au point et adaptés aux principes de fonctionnement de ces appareils.

Ainsi on peut mesurer les tailles des cellules (après lyse différentielle de leur cytoplasme) soit par mesure de variations de résistivité soit par mesure de diffraction optique ; on peut aussi effectuer des colorations (enzymatiques ou non) spécifiques des différents types cellulaires et mesurer les tailles et densités optiques des cellules à différentes longueurs d'onde.

Un des problèmes posés par l'automatisation est la confusion possible entre les erythrocytes et certains leucocytes de petite taille, artefact qui n'apparaissait pas à l'observation au microscope. Il faut donc assurer la lyse totale des érythrocytes avant le passage de l'échantillon sanguin dans l'appareil, tout en préservant l'intégrité des leucocytes. De plus, le mélange réactionnel doit comprendre un ou plusieurs réactifs qui faciliteront la discrimination des différents types de leucocytes et leur regroupement en zones bien distinctes sur un histogramme.

Ainsi le brevet WO 8505684 (Coulter Electronics Inc) décrit un ensemble de réactifs qui assurent, en deux étapes, une lyse spécifique des érythrocytes par la saponine, puis, une fixation (et par ce fait une protection) des leucocytes par le glutaraldéhyde. De plus, la discrimination des différents types de leucocytes est favorisée par l'addition d'un réactif comme le phénoxy-2-éthanol ; certaines populations peuvent encore être spécifiquement colorées et identifiées par fluorescence. Ce procédé efficace et fiable mais complexe est particulièrement adapté à un appareil de type Coulter counter® tel que décrit dans le brevet US 3,502,974.

D'autres méthodes, décrites par exemple dans le brevet US 3,740,143, particulièrement adaptées aux appareils Technicon®, mettent en jeu plusieurs réactions effectuées en parallèle et révélant chacune un type cellulaire, ces réactions spécifiques étant en outre précédées ou suivies d'un traitement de lyse des érythrocytes et éventuellement d'un traitement de fixation des leucocytes. Les réactions spécifiques comprennent des colorations histologiques classiques (comme le "rouge neutre" pour les basophiles) et des colorations enzymatiques (comme la révélation du contenu en peroxydases des éosinophiles et neutrophiles par le chloronaphtol ou du contenu en lipases des monocytes par le butyrate de naphtol).

D'une manière générale, des analyses détaillées et fiables ne sont réalisées que par des appareils fort complexes et par des méthodes qui comprennent plusieurs étapes successives, opérées avec des réactifs différents. Leur complexité rend le coût de ces analyses fort élevé, et, pour des diagnostics de routine, il est toujours souhaitable de simplifier le procédé, tout en gardant un même pouvoir de discrimination et un même degré de fiabilité.

Ainsi la Demanderesse a mis au point une composition qui permet, en une seule réaction, d'effectuer la lyse des érythrocytes, la fixation-protection des leucocytes et la coloration des éosinophiles et d'assurer une très bonne séparation des différentes sous-populations leucocytaires sur un histogramme. Le mode d'utilisation de la composition selon l'invention est particulièrement rapide et est adapté à n'importe quel type d'appareil de comptage automatique.

Plus particulièrement, le réactif selon l'invention comprend, d'une part, au moins un agent de lyse des érythrocytes et, de préférence, une saponine et du dodécyl sulfate de sodium (SDS), d'autre part, un agent capable de colorer différentiellement les diverses populations leucocytaires et, en particulier, le Noir chlorazol à une concentration comprise entre 50 et 650 mg/l. Les autres contituants du réactif qui sont destinés à préserver l'intégrité et la morphologie cellulaires naturelles et à améliorer leur discrimination en détection optique, comprennent au-moins un sel physiologique pour maintenir le pH entre 7,0 et 12,0, un sel d'ammonium tertiaire ou quaternaire, un alcool primaire, secondaire ou tertiaire, un agent tensioactif, un préservateur des structures cellulalres et un alkylèneglycol.

Le Noir chlorazol (aussi appelé Noir Erié ou Noir formique) est un réactif classique, utilisé depuis 1937 en histologie végétale (Cannon, H.G, Nature, 139, 549) et utilisé pour la coloration spécifique des éosinophiles, pour l'observation au microscope. (Ce réactif et son mode d'utilisation ont été décrits par M. Piette, Ann. Biol. Clin. 1961, 19, 729-734 et K.Lawrence, Am. J. Clin. Pathol. 1981, 76, 810-812.).

Les conditions habituelles d'utilisation du Noir chlorazol (650 mg/l) ne sont pas du tout adaptées à une détection en appareil automatique à photodiode, les éosinophiles étant beaucoup trop noirs et les autres cellules apparaissant totalement transparentes. De nouvelles conditions d'utilisation ont été mises au point, à la fois en diminuant la concentration du réactif et en augmentant la température à laquelle la réaction est opérée sur l'échantillon sanguin.

Ainsi le réactif selon l'invention contient de préférence une concentration de Noir chlorazol inférieure à 100 mg/l. La Demanderesse a observé que, dans ces conditions, les éosinophiles sont nettement colorés et, en outre, les autres granulocytes sont fixés et clairement discriminés. Une légère coloration de toutes les populations leucocytaires apparait mais elle s'efface si on prolonge la durée de réaction.

En cherchant le meilleur équilibre des divers constituants du réactif, la Demanderesse a constaté que l'addition de glutaraldéhyde en solution aqueuse améliorait la discrimination des tailles des cellules, quand la mesure est réalisée par résistivité. Toutefois le glutaraldéhyde augmente la taille des stromas résultant de la lyse des érythrocytes. Cet artefact est avantageusement corrigé par l'addition de SDS qui accélère la destruction des érythrocytes, à des concentrations qui n'abiment pas la morphologie des leucocytes. En outre, l'addition d'un sel d'ammonium tertiaire ou quaternaire améliore encore la discrimination entre les sous-populations cellulaires et participe également à la destruction des stromas érythrocytaires. On utilisera avantageusement le chlorure de dodécyltriméthylammonium. On obtient des résultats équivalents avec les bromures et les iodures ; de même on peut utiliser les sels tertiaires ou quaternaires dont les radicaux alcoyles préférés sont les C₁₂ et C₁₄. Ainsi on choisira un halogénure (Cl, Br ou I), de dodécyldi(ou tri)méthylammonium ou un tétradécyldi(ou tri)méthylammonium, seul ou en combinaison.

Le réactif selon l'invention contient également un agent tensioactif choisi par exemple dans la famille des esters de polyoxyéthylène sorbitane et plus particulièrement le monolaurate de polyoxyéthylène sorbitane (Tween 20®), et le monooléate de polyoxyéthylène sorbitane (Tween 80 ®).

D'autre part l'addition d'un alkylèneglycol qui exerce également un effet préservateur sur les structures cellulaires, a surtout un effet favorable pour la lecture optique en limitant la diffraction du milieu.

Ainsi selon un mode préféré de réalisation de l'invention, le réactif comprend les constituants suivants :
- une saponine, à une concentration comprise entre 100 et 700 mg/l,
- dù SDS, à une concentration comprise entre 100 et 400 mg/l,
- du Noir chlorazol, de préférence à une concentration inférieure à 100 mg/l,
- un sel ou un mélange de sels physiologiques comprenant de 1,3 à 9,5 g/l de chlorure de sodium, de 15 à 20 g/l de sulfate de sodium, de 400 à 700 mg/l de carbonate de sodium,
- au moins un sel d'ammonium tertiaire ou quaternaire, dans un rapport final de 0,03 à 0,10 % en volume,
- un alcool primaire, secondaire ou tertiaire et, de préférence, l'alcool isopropylique dans un rapport final de 2 à 13 % en volume,
- un ester de polyoxyéthylène sorbitane en solution aqueuse, dans un rapport final de 0,2 à 2 96 en volume,
- du glutaraldéhyde ou du formaldéhyde en solution aqueuse, dans un rapport final de 0,1 à 0,8 % en volume,
- de l'éthylèneglycol ou du propylèneglycol, dans un rapport final de 2 à 13 % en volume.

L'invention concerne également une méthode d'utilisation de ce réactif pour la détermination en appareil de mesure automatique à cytométrie de flux d'au moins une sous-population leucocytaire. La méthode selon l'invention comprend la mise en contact de l'échantillon sanguin à étudier et du réactif, dans une enceinte thermostatée, à une température comprise entre 20 et 80 ° C et, de préférence, entre 40 et 65°, pendant une durée inférieure à 20 secondes.

La méthode comprend ensuite la détection et la discrimination des leucocytes par mesure des variations d'absorbance ou de résistivité ou la combinaison des deux, par un appareil de détection approprié et l'enregistrement de ces mesures sous forme de matrices ou d'histogrammes permettant de visualiser des contours représentatifs de la répartition des sous-populations leucocytaires.

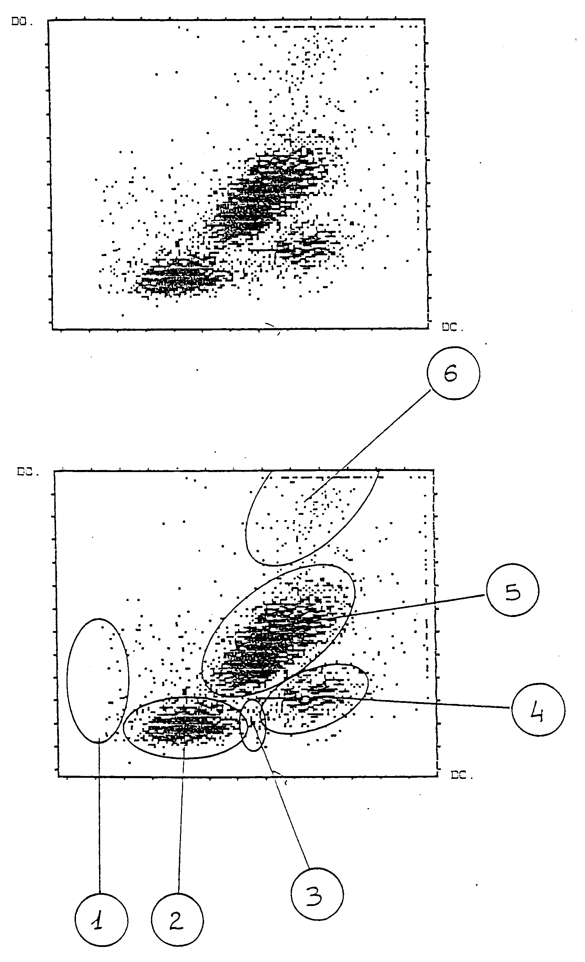

La figure 1 est un histogramme représentatif, obtenu par la mise en oeuvre de la méthode selon l'invention, sur un échantillon de sang normal. Sur l'histogramme inférieur (identique à l'histogramme supérieur) on a délimité les zones correspondant aux différentes populations :
1- bruit de fond (stromas, plaquettes, déchets)
2- lymphocytes
3- polynucléaires basophiles
4- monocytes
5- polynucléaires neutrophiles
6- polynucléaires éosinophiles (DO = densité optique ; DC = résistivité en courant continu)

L'exemple suivant illustre un mode de réalisation de l'invention sans toufefois en restreindre la portée.

### Exemple

Les concentrations suivantes des divers constituants sont calculées pour la préparation d'un litre de réactif :
- saponine 650 mg
- SDS 200 mg
- Noir Chlorazol 65 mg
- chlorure de sodium 5,144 g
- sulfate de sodium 17,4 g
- carbonate de sodium 650 mg
- sel d'ammonium 0,3 ml
- alcool isopropylique 130 ml
- Tween® 80 13 ml
- glutaraldéhyde 4 ml
- propylèneglycol 130 ml
- eau distillée, jusqu'à ajustement du volume final à 1 litre.

Le réactif est utilisé par exemple dans un appareil de détection tel que décrit dans FR-A-2 653 885 au nom de la Demanderesse mais il pourrait, de la même façon, être utilisé dans tout autre type de compteur automatique des populations leucocytaires.

L'appareil est réglé pour mettre en contact un échantillon de 25 »l de sang total et 1 ml de réactif. Ce mélange est réalisé dans une enceinte thermostatée à une température co mprise entre 40 et 65° C.

Un histogramme représentatif d'un échantillon de sang normal est présenté sur la figure 1.

## Revendications

1. Réactif pour la détermination en appareil de mesure automatique en cytométrie de flux d'au moins une sous-population leucocytaire, caractérisé en ce qu'il comprend les constituants suivants :
- au moins un agent de lyse des érythrocytes
- du Noir chlorazol
- au moins un sel physiologique
- au moins un sel d'ammonium tertiaire ou quaternaire
- un alcool
- un agent tensioactif
- un préservateur
- un alkylèneglycol

2. Réactif selon la revendication 1 caractérisé en ce que l'agent de lyse des érythrocytes est choisi parmi les saponines et le dodécyl sulfate de sodium, seul ou en combinaison.

3. Réactif selon la revendication 2 caractérisé en ce que la concentration de saponine est comprise entre 100 et 700 mg par litre de réactif.

4. Réactif selon la revendication 2 caractérisé en ce que la concentration de dodécyl sulfate de sodium est comprise entre 100 et 400 mg par litre de réactif.

5. Réactif selon l'une quelconque des revendications 1 à 4 caractérisé en ce que la concentration de Noir chlorazol est comprise entre 50 et 650 mg par litre de réactif .

6. Réactif selon la revendication 5 caractérisé en ce que la concentration de Noir chlorazol est inférieure à 100 mg/l.

7. Réactif selon l'une quelconque des revendications 1 à 6 caractérisé en ce qu'il comprend un sel physiologique choisi parmi le chlorure de sodium, le sulfate de sodium, le carbonate de sodium, seul ou en combinaison, pour maintenir le pH entre 7,0 et 12,0.

8. Réactif selon la revendication 7 caractérisé en ce que la concentration de chlorure de sodium est comprise entre 1,3 et 9,5 g par litre de réactif.

9. Réactif selon la revendication 7 caractérisé en ce que la concentration de sulfate de sodium est comprise entre 15 et 20 g par litre de réactif.

10. Réactif selon la revendication 7 caractérisé en ce que la concentration en carbonate de sodium est comprise entre 400 et 700 mg par litre de réactif.

11. Réactif selon l'une quelconque des revendications 1 à 10 caractérisé en ce qu'il comprend au moins un sel d'ammonium tertiaire ou quaternaire dans un rapport final de 0,03 à 0,10 % en volume du réactif.

12. Réactif selon l'une quelconque des revendications 1 à 11 caractérisé en ce que l'alcool est choisi parmi les alcools primaires, secondaires et tertiaires, dans un rapport final de 2 à 13 % en volume du réactif.

13. Réactif selon l'une quelconque des revendications 1 à 12 caractérisé en ce que l'agent tensioactif est un ester de polyoxyéthylène sorbitane en solution aqueuse, dans un rapport final de 0,2 à 2 % en volume du réactif.

14. Réactif selon l'une quelconque des revendications 1 à 13 caractérisé en ce que le préservateur est choisi parmi le glutaraldéhyde et le formaldéhyde, en solution aqueuse, dans un rapport final de 0,1 à 0,8 % en volume du réactif.

15. Réactif selon l'une quelconque des revendications l à 14 caractérisé en ce que l'alkylèneglycol est choisi parmi l'éthylèneglycol et le propylèneglycol, dans un rapport final de 2 à 13 % en volume du réactif.

16. Méthode de détermination en appareil de mesure automatique en cytométrie de flux d'au-moins une sous-population leucocytaire, caractérisée en ce qu'elle comprend :
- la mise en contact d'un échantillon de sang avec le réactif selon l'une quelconque des revendications 1 à 15, dans une enceinte thermostatée,
- la détection et la discrimination des leucocytes par mesure des variations de résistivité ou d'absorbance ou la combinaison des deux,
- l'enregistrement de ces mesures sous forme de matrices ou d'histrogrammes pemettant de visualiser des contours représentatifs de la répartition des sous-populations leucocytaires.

17. Méthode selon la revendication 16 caractérisée en ce que la mise en contact du sang et du réactif est effectuée à une température comprise entre 20 et 80°C et pendant une durée inférieure à 20 secondes.

## Patentansprüche

1. Reagenz für die durchflußcytometrische Bestimmung von wenigstens einer Leukocyten-Subpopulation in einem automatischen Meßgerät, dadurch gekennzeichnet, daß das Reagenz folgende Bestandteile enthält:
- wenigstens ein Lösungsmittel für Erythrocyten
- Chlorazol Schwarz
- wenigstens ein physiologisches Salz
- wenigstens ein tertiäres oder quartäres Ammonium-Salz
- einen Alkohol
- einen grenzflächenaktiven Stoff
- ein Schutzmittel
- ein Alkylenglykol.

2. Reagenz nach Anspruch 1, dadurch gekennzeichnet, daß das Lösungsmittel für Erythrocyten allein oder in Kombination ausgewählt ist aus den Saponinen und dem Natriumdodekylsulfat.

3. Reagenz nach Anspruch 2, dadurch gekennzeichnet, daß die Konzentration des Saponins zwischen 100 und 700 mg je Liter Reagenz beträgt.

4. Reagenz nach Anspruch 2, dadurch gekennzeichnet, daß die Konzentration des Natriumdodekylsulfats zwischen 100 und 400 mg je Liter Reagenz beträgt.

5. Reagenz nach einem der Ansprüche 1 bis 4, dadurch gekennzeichnet, daß die Konzentration des Chlorazol Schwarzes zwischen 50 und 650 mg je Liter Reagenz beträgt.

6. Reagenz nach Anspruch 5, dadurch gekennzeichnet, daß die Konzentration des Chlorazol Schwarzes kleiner als 100 mg/l ist.

7. Reagenz nach einem der Ansprüche 1 bis 6, dadurch gekennzeichnet, daß ein physiologisches Salz allein oder in Kombination ausgesucht aus Natriumchlorid, Natriumsulfat und Natriumcarbonat enthalten ist, um einen pH-Wert zwischen 7,0 und 12,0 zu erhalten.

8. Reagenz nach Anspruch 7, dadurch gekennzeichnet, daß die Konzentration des Natriumchlorids zwischen 1,3 und 9,5 g je Liter Reagenz beträgt.

9. Reagenz nach Anspruch 7, dadurch gekennzeichnet, daß die Konzentration des Natriumsulfats zwischen 15 und 20 g je Liter Reagenz beträgt.

10. Reagenz nach Anspruch 7, dadurch gekennzeichnet, daß die Konzentration des Natriumcarbonats zwischen 400 und 700 mg je Liter Reagenz beträgt.

11. Reagenz nach einem der Ansprüche 1 bis 10, dadurch gekennzeichnet, daß ein tertiäres oder quartäres Ammonium-Salz in einem Endverhältnis von 0,03 bis 0,10 Volumenprozent des Reagenzes enthalten ist.

12. Reagenz nach einem der Ansprüche 1 bis 11, dadurch gekennzeichnet, daß der Alkohol ausgewählt ist aus primären, sekundären und tertiären Alkoholen und in einem Endverhältnis von 2 bis 13 Volumenprozent des Reagenzes enthalten ist.

13. Reagenz nach einem der Ansprüche 1 bis 12, dadurch gekennzeichnet, daß der grenzflächenaktive Stoff ein Polyoxyethylen-Sorbitanester in wäßriger Lösung ist und in einem Endverhältnis von 0,2 bis 2,0 Volumenprozent des Reagenzes enthalten ist.

14. Reagenz nach einem der Ansprüche 1 bis 13, dadurch gekennzeichnet, daß das Schutzmittel ausgewählt ist aus dem Glutaraldehyd und dem Formaldehyd in wäßriger Lösung und in einem Endverhältnis von 0,1 bis 0,8 Volumenprozent des Reagenzes enthalten ist.

15. Reagenz nach einem der Ansprüche 1 bis 14, dadurch gekennzeichnet, daß das Alkylenglykol ausgesucht ist aus dem Ethylenglykol und dem Propylenglykol und in einem Endverhältnis von 2 bis 13 Volumenprozent des Reagenzes enthalten ist.

16. Verfahren zur durchflußcytometrischen Bestimmung von wenigstens einer Leukocyten-Subpopulation in einem automatischen Meßgerät, dadurch gekennzeichnet, daß das Verfahren die Schritte enthält:
- das Inberührungbringen einer Blutprobe mit dem Reagenz gemäß einem der Ansprüche 1 bis 15 in einem temperaturgeregelten Behälter,
- das Erfassen und das Unterscheiden der Leukocyten durch Messen der Veränderungen des spezifischen Widerstandes oder des Absorbtionskoeffizienten oder der Kombination der beiden,
- das Eintragen der Messungen unter Bildung von Matrizen oder Histogrammen, die eine Darstellung der repräsentativen Konturen der Verteilung der Leukocyten-Subpopulationen ermöglichen.

17. Verfahren nach Anspruch 16, dadurch gekennzeichnet, daß das Inberührungbringen des Blutes mit dem Reagenz bei einer Temperatur zwischen 20 und 80°C während einer Dauer von weniger als 20 Sekunden durchgeführt wird.

## Claims

1. Reagent for determining, in an automatic flow cytometry measuring apparatus, at least one leukocyte subpopulation, characterized in that it comprises the following constituents:
- at least one erythrocyte-lysing agent
- chlorazole black
- at least one physiological salt
- at least one tertiary or quaternary ammonium salt
- an alcohol
- a surfactant
- a preservative
- an alkylene glycol

2. Reagent according to Claim 1, characterized in that the erythrocyte-lysing agent is chosen from saponins and sodium dodecyl sulphate, alone or in combination.

3. Reagent according to Claim 2, characterized in that the saponin concentration is between 100 and 700 mg per litre of reagent.

4. Reagent according to Claim 2, characterized in that the sodium dodecyl sulphate concentration is between 100 and 400 mg per litre of reagent.

5. Reagent according to any one of Claims 1 to 4, characterized in that the chlorazole black concentration is between 50 and 650 mg per litre of reagent.

6. Reagent according to Claim 5, characterized in that the chlorazole black concentration is less than 100 mg/l.

7. Reagent according to any one of Claims 1 to 6, characterized in that it comprises a physiological salt chosen from sodium chloride, sodium sulphate, and sodium carbonate, alone or in combination, in order to maintain the pH between 7.0 and 12.0.

8. Reagent according to Claim 7, characterized in that the sodium chloride concentration is between 1.3 and 9.5 g per litre of reagent.

9. Reagent according to Claim 7, characterized in that the sodium sulphate concentration is between 15 and 20 g per litre of reagent.

10. Reagent according to Claim 7, characterized in that the sodium carbonate concentration is between 400 and 700 mg per litre of reagent.

11. Reagent according to any one of Claims 1 to 10, characterized in that it comprises at least one tertiary or quaternary ammonium salt in a final ratio of 0.03 to 0.10% by volume of the reagent.

12. Reagent according to any one of Claims 1 to 11, characterized in that the alcohol is chosen from primary, secondary and tertiary alcohols, in a final ratio of 2 to 13% by volume of the reagent.

13. Reagent according to any one of Claims 1 to 12, characterized in that the surfactant is a polyoxyethylene sorbitan ester in aqueous solution, in a final ratio of 0.2 to 2% by volume of the reagent.

14. Reagent according to any one of Claims 1 to 13, characterized in that the preservative is chosen from glutaraldehyde and formaldehyde, in aqueous solution, in a final ratio of 0.1 to 0.8% by volume of the reagent.

15. Reagent according to any one of Claims 1 to 14, characterized in that the alkylene glycol is chosen from ethylene glycol and propylene glycol, in a final ratio of 2 to 13% by volume of the reagent.

16. Method of determining, in a flow cytometry automatic measuring apparatus, at least one leukocyte subpopulation, characterized in that it comprises:
- placing a blood sample in contact with the reagent according to any one of Claims 1 to 15, in a thermostatted chamber,
- detecting and discriminating between the leukocytes by measuring the variations in resistivity or absorbance, or the combination of the two,
- recording these measurements in the form of matrixes or histograms which make it possible to visualize contours representative of the distribution of the leukocyte subpopulations.

17. Method according to Claim 16, characterized in that the placing of the blood in contact with the reagent is carried out at a temperature of between 20 and 80°C and for a period of less than 20 seconds.
